(19) Europäisches Patentamt
European Patent Office
Office européen des brevets

(11) EP 1 002 523 B1

(12) # FASCICULE DE BREVET EUROPEEN

(45) Date de publication et mention de la délivrance du brevet:
**03.03.2004 Bulletin 2004/10**

(51) Int Cl.⁷: **A61K 7/42**

(21) Numéro de dépôt: **99401898.4**

(22) Date de dépôt: **26.07.1999**

(54) **Compositions cosmétiques photoprotectrices**

Kosmetische Sonnenschutzmittel

Cosmetic sunscreening compositions

(84) Etats contractants désignés:
**AT BE CH CY DE DK ES FI FR GB GR IE IT LI LU MC NL PT SE**

(30) Priorité: **25.09.1998 FR 9812042**

(43) Date de publication de la demande:
**24.05.2000 Bulletin 2000/21**

(73) Titulaire: **L'OREAL**
**75008 Paris (FR)**

(72) Inventeurs:
- **Hansenne, Isabelle**
**75017 Paris (FR)**
- **Josso, Martin**
**75005 Paris (FR)**
- **De Chabannes, Karine**
**45000 Orleans (FR)**

(74) Mandataire: **Miszputen, Laurent**
**L'OREAL - D.I.P.I.**
**25-29 Quai Aulagnier**
**92600 Asnières (FR)**

(56) Documents cités:
**EP-A- 0 711 779** **EP-A- 0 742 003**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 002 523 B1

## Description

La présente invention concerne de nouvelles compositions cosmétiques à usage topique plus particulièrement destinées à la photoprotection de la peau et/ou des cheveux contre le rayonnement ultraviolet (compositions ci-après dénommées plus simplement compositions antisolaires), ainsi que leur utilisation dans l'application cosmétique susmentionnée. Plus précisément encore, elle concerne des compositions antisolaires comprenant, dans un support cosmétiquement acceptable, une association d'au moins deux filtres lipophiles particuliers, l'un des filtres possédant des propriétés solubilisantes vis à vis de l'autre filtre. L'invention concerne par ailleurs un procédé de solubilisation d'un ou deux filtres lipophiles solides particuliers au moyen d'un ou deux types de filtres lipophiles liquides spécifiques.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Pour diverses raisons liées en particulier à un meilleur confort d'utilisation (douceur, émollience, facilité d'application et autres), les compositions antisolaires actuelles se présentent le plus souvent sous la forme d'une émulsion de type huile-dans-eau (c'est à dire un support consmétiquement acceptable constitué d'une phase continue dispersante aqueuse et d'une phase discontinue dispersée huileuse) qui contient, à des concentrations diverses, un ou plusieurs filtres organiques classiques capables d'absorber selectivement les rayonnements UV nocifs, ces filtres étant sélectionnés en fonction de l'indice de protection recherché (l'indice de protection (IP) s'exprimant mathématiquement par !e rapport du temps d'irradiation nécessaire pour atteindre le seuil érythématogène avec le filtre UV au temps nécessaire pour atteindre le seuil érythématogène sans filtre UV). Se!on leur caractère lipophile ou au contraire hydrophile, ces filtres peuvent se répartir, respectivement, soit dans la phase grasse, soit dans la phase aqueuse, de la composition finale.

Il s'avère que des filtres organiques UV lipophiles particulièrement intéressants en cosmétique antisolaire et fortement actifs dans l'UV-A et dans l'UV-B ont été décrits dans les demandes EP-A-0392883 ; EP-A-0660701 ; EP-A-0708108 ; EP-A-0711778 ; EP-A-711779. Il s'agit de silanes ou de polyorganosiloxanes à fonction benzotriazole. Ils présentent la particularité mais aussi le désavantage d'être solides à température ambiante. De ce fait, leur utilisation dans une composition cosmétique antisolaire implique certaines contraintes au niveau de leur formulation et de leur mise en oeuvre, en particulier lorsqu'il s'agit de trouver des solvants permettant de les solubiliser correctement. A cet égard, on fait aujourd'hui le plus souvent appel à des huiles comme des esters et plus particulièrement des benzoates d'alkyles en $C_{12}$-$C_{15}$ ("FINSOLV TN" de chez Finetex), ou à des triglycérides et notamment des triglycérides d'acides gras en $C_8$-$C_{12}$ ("MIGLYOL 812" de chez Hüls), ou encore à des monoalcools ou des polyols tels que l'éthanol, ainsi qu'à leurs mélanges. Ces produits, bien que possédant des propriétés solubilisantes vis à vis des filtres susmentionnés, présentent néanmoins pour inconvénient de n'avoir aucune activité propre dans le domaine de la filtration du rayonnement UV, tant UV-A qu'UV-B.

Or, la Demanderesse vient maintenant de découvrir, de façon inattendue et surprenante, que les filtres UV du type dérivé de l'acide cinnamique et/ou les filtres UV du type dérivé de l'acide salicylique constituent des solvants particulièrement remarquables pour les filtres solides susmentionnés, à savoir les dérivés silicés à fonction benzotriazole. On notera que les dérivés de l'acide cinnamique et les dérivés de l'acide salicylique sont des filtres lipophiles liquides déjà connus pour leur activité dans l'UV-B, mais leurs propriétés solubilisantes vis à vis des filtres solides précités n'ont, quant à elles, jamais été décrites. L'intérêt de la présente découverte est ainsi double, en ce sens qu'il est maintenant possible d'une part d'effectuer la solubilisation d'un dérivé silicié à fonction benzotrazole ou d'un mélange de ceux-ci, par un solvant autre que ceux antérieurement connus, ce qui est toujours intéressant en soi, et d'autre part, d'augmenter la quantité de filtres et d'obtenir dans la composition antisolaire finale une augmentation substantielle du niveau de protection attachée à cette dernière.

Toutes ces découvertes sont à la base de la présente invention.

Ainsi, conformément à l'un des objets de la présente invention, il est maintenant proposé de nouvelles compositions cosmétiques, en particulier antisolaires, qui sont caractérisées par le fait qu'elles comprennent, dans un support cosmétiquement acceptable :

(i) un système filtrant solubilisé constitué d'au moins un dérivé silicié à fonction benzotriazole répondant à l'une des formules (5) ou (6) définies ci-dessous ;
(ii) un système filtrant solubilisant constitué :

- d'au moins un composé (A) choisi dans le groupe formé par le 4-méthoxy cinnamate d'isopentyle, le 4-méthoxy cinnamate de 2-éthylhexyle, le diisopropyl cinnamate de méthyle, le 4-méthoxy cinnamate d'isoamyle, le 4-méthoxy cinnamate de diéthanolamine et/ou
- au moins un composé (B) choisi dans le groupe constitué par le salicylate d'homomenthyle, le salicylate de 2-éthylhexyle, le salicylate de triéthanolamine, le salicylate de 4-isopropylbenzyle et les composés ou les mélanges de composés répondant à la formule (I) suivante :

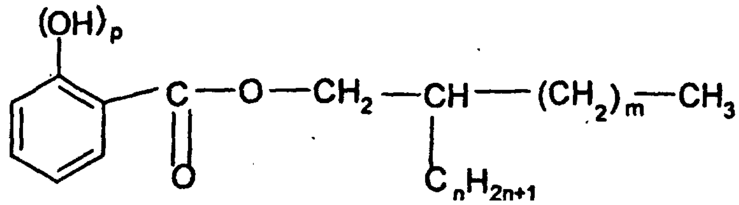

(I)

dans laquelle m vaut 5, 7 ou 9 ; n vaut 4, 6 ou 8 ; p vaut 0 ou 1 ; sous réserve.que lorsque p vaut 0 alors m vaut 5 ou 7 et n vaut 4 ou 6 ;
ledit système filtrant solubilisant étant présent en une quantité suffisante pour solubiliser à lui seul l'ensemble dudit système filtrant solubilisé.

La présente invention a également pour objet l'utilisation des compositions ci-dessus pour la fabrication de compositions cosmétiques destinées à la protection de la peau et/ou des cheveux contre le rayonnement ultraviolet, en particulier le rayonnement solaire.

Un autre objet encore de la présente invention réside dans un procédé de traitement cosmétique pour la protection de la peau et/ou des cheveux contre le rayonnement ultraviolet, en particulier le rayonnement solaire, et qui consiste essentiellement à appliquer sur ces derniers une quantité efficace d'une composition conforme à l'invention.

La présente invention a également enfin pour objet l'utilisation pour la fabrication d'une composition cosmétique destinée à la protection de la peau et/ou des cheveux contre les radiations UV, d'au moins un composé (A) tel que défini précédemment et/ou d'au moins un composé (B) tel que défini précédemment comme système filtrant solubilisant permettant dans une quantité suffisante de solubiliser à lui seul au moins un filtre UV solide du type dérivé silicié à fonction benzatriazole présent dans ladite composition.

D'autres caractéristiques, aspects et avantages de la présente invention apparaitront à la lecture de la description détaillée qui va suivre.

Les dérivés siliciés à fonction benzotriazole utilisés dans la présente invention sont de préférence des silanes ou des siloxanes à fonction benzotriazole comprenant au moins une unité de formule (1) suivante :

$$O_{(3-a)/2}Si(R_7)_a\text{-}G \qquad (1)$$

dans laquelle :

- $R_7$ représente un radical alkyle en $C_1$-$C_{10}$ éventuellement halogéné ou un radical phényle ou un radical triméthylsilyloxy,
- a est un nombre entier choisi entre 0 et 3 inclusivement,
- et le symbole G désigne un radical monovalent lié directement à un atome de silicium, et qui répond à la formule (2) suivante :

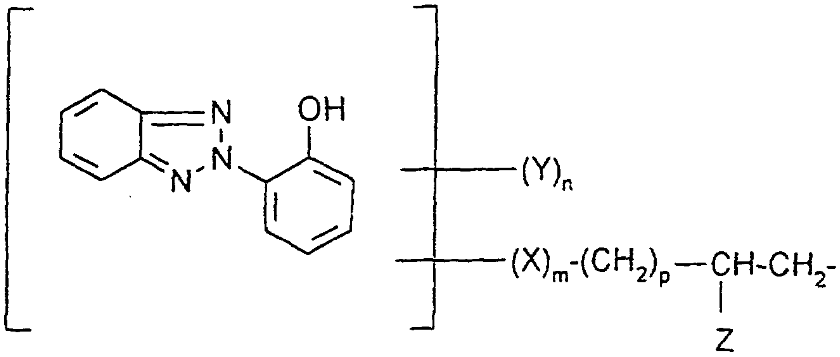

(2)

dans laquelle :

- Y, identiques ou différents, sont choisis parmi les radicaux alkyles en $C_1$-$C_8$, les halogènes et les radicaux alkoxy en $C_1$-$C_4$ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z représente l'hydrogène ou un radical alkyle en $C_1$-$C_4$,
- n est un nombre entier compris entre 0 et 3 inclusivement,
- m est 0 ou 1,
- p représente un nombre entier compris entre 1 et 10, inclusivement.

Ces composés sont notamment décrits dans les demandes de brevet EP-A-0392883 ; EP-A-0660701 ; EP-A-0708108 ; EP-A-0711778 ; EP-A-711779.

De préférence, les dérivés siliciés utilisés dans le cadre de la présente invention appartiennent à la famille générale des silicones benzotriazoles qui est décrite notamment dans EP-A-0660701 .

Une famille de silicones benzotriazoles convenant particulièrement bien à la réalisation de la présente invention est celle regroupant les composés répondant aux formules (5) ou (6) suivantes :

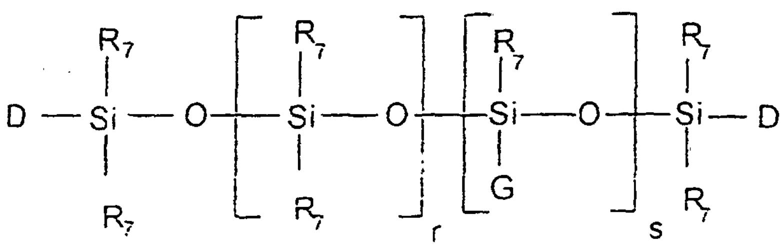

(5)

ou

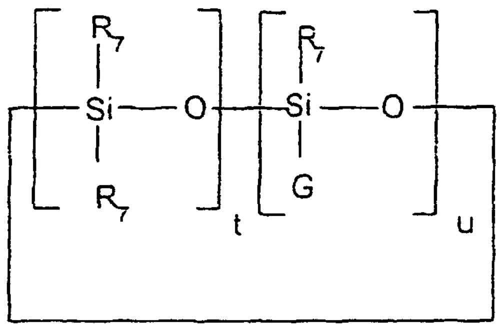

(6)

dans lesquelles :

- $R_7$, identiques ou différents, sont choisis parmi les radicaux alkyles en $C_1$-$C_{10}$, phényle, trifluoro-3,3,3 propyle et triméthylsilyloxy, au moins 80% en nombre des radicaux $R_7$ étant méthyle,
- D, identiques ou différents sont choisis parmi les radicaux $R_7$ et le radical G,
- r est un nombre entier compris entre 0 et 50 inclusivement, et s est un nombre entier compris entre 0 et 20 inclusivement, et si s = 0, au moins l'un des deux symboles D désigne G,
- u est un nombre entier compris entre 1 et 6 inclusivement, et t est un nombre entier compris entre 0 et 10 inclusivement, étant entendu que t + u est égal ou supérieur à 3,
- et le symbole G répond à la formule (2) ci-dessus.

Comme cela ressort de la formule (2) donnée ci-dessus, l'accrochage du chaînon - $(X)_m$-$(CH_2)_p$-CH(Z)-$CH_2$- sur le motif benzotriazole, qui assure donc le raccordement dudit motif benzotriazole à l'atome de silicium de la chaîne siliconée, peut, selon la présente invention, se faire dans toutes les positions disponibles offertes par les deux noyaux aromatiques du benzotriazoie :

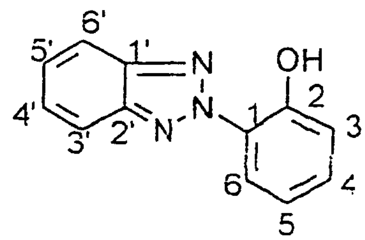

De préférence, cet accrochage se fait en position 3. 4, 5 (noyau aromatique portant la fonction hydroxy) ou 4' (noyau benzénique adjacent le cycle triazolé), et encore plus préférentiellement en position 3, 4 ou 5 Dans une forme préférée de réalisation de l'invention, l'accrochage se fait en position 3.

De même, l'accrochage du ou des motifs substituants Y peut se faire dans toutes les autres positions disponibles au sein du benzotriazole. Toutefois, de préférence, cet accrochage se fait en position 3, 4, 4', 5 et/ou 6. Dans une forme préférée de réalisation de l'invention, l'accrochage du motif Y se fait en position 5.

Dans les formules (5) et (6) ci-dessus, les radicaux alkyle peuvent être linéaires ou ramifiés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et ter.-octyle. Les radicaux alkyle $R_7$ préférés selon l'invention sont les radicaux méthyle, éthyle, propyle, n-butyle, n-octyle et éthyl-2 hexyle. Encore plus préférentiellement, les radicaux $R_7$ sont tous des radicaux méthyle.

Parmi les composés de formules (5) ou (6) ci-dessus, on préfère mettre en oeuvre ceux répondant à la formule (5) c'est-à-dire des diorganosiloxanes à chaîne courte linéaire.

Parmi les composés de formules (5) ci-dessus, on préfère mettre en oeuvre ceux pour lesquels les radicaux D sont tous les deux des radicaux $R_7$

Parmi les diorganosiloxanes linéaires rentrant dans le cadre de la présente invention, on préfère plus particulièrement les dérivés statistiques ou bien définis à blocs présentant au moins l'une, et encore plus préférentiellement l'ensemble, des caractéristiques suivantes :

- D est un radical $R_7$
- $R_7$ est alkyle et encore plus préférentiellement est méthyle,
- r est compris entre 0 et 15 inclusivement ; s est compris entre 1 et 10 inclusivement,
- n est non nul, et de préférence égal à 1, et Y est alors choisi parmi méthyle, tert.-butyle ou alcoxy en $C_1$-$C_4$.
- Z est hydrogène ou méthyle,
- m=0, ou [m=1 et X=O]
- p est égal à 1.

Une famille de silicones benzotriazoles convenant particulièrement bien à l'invention est celle définie par la formule générale (7) suivante :

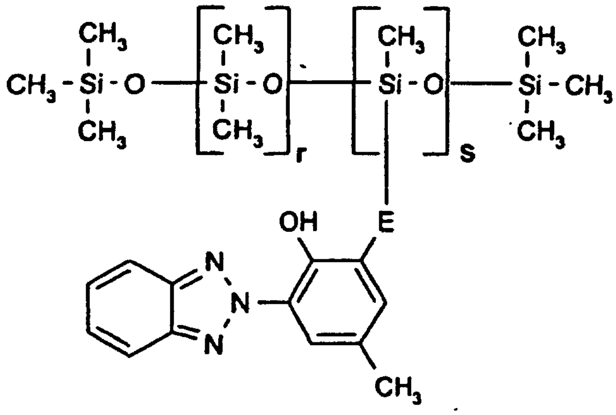

(7)

avec

$$0 \leq r \leq 10,$$

$$1 \leq s \leq 10,$$

et où E représente le radical divalent :

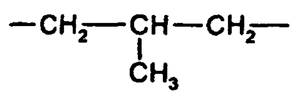

Dans une forme particulièrement préférée de réalisation de l'invention, la silicone benzotriazole est le composé (appelé composé (a) dans la suite du texte) répondant à la formule suivante :

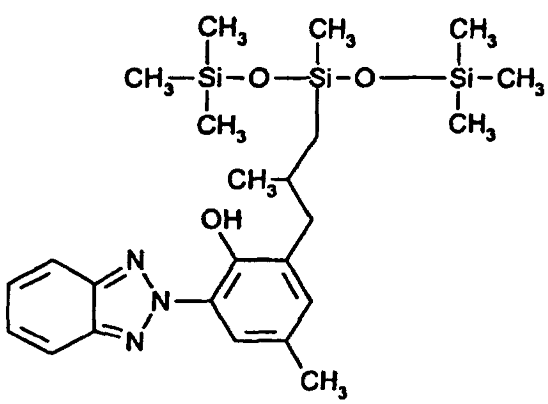

**composé (a)**

Des procédés convenant à la préparation des produits de formule (1), (5), (6) et (7) ci-dessus sont notamment décrits dans les brevets américains US 3, 220, 972, US 3, 697, 473, US 4, 340, 709, US 4, 316, 033, US 4, 328, 346 et dans les demandes de brevet EP-A-0 392 883 et EP-A-0 742 003.

Le dérivé silicié à fonction benzotriazole peut être présent dans les compositions selon l'invention à des teneurs allant de 0,1 à 20%, de préférence allant de 0,2 à15%, en poids, toujours par rapport au poids total de la composition. Selon une caractéristique essentielle de la présente invention, ces composés, pris seuls ou en mélanges, doivent se présenter dans la composition finale sous une forme totalement, ou substantiellement totalement, solubilisée.

Parmi les composés (A) mentionnés ci-dessus, on préfère tout particulièrement, selon la présente invention,

mettre en oeuvre le p-méthoxycinnamate de 2-éthylhexyle, vendu notamment sous le nom commercial « PARSOL MCX » par la Société GIVAUDAN ; ce filtre répondant donc à la formule développée suivante:

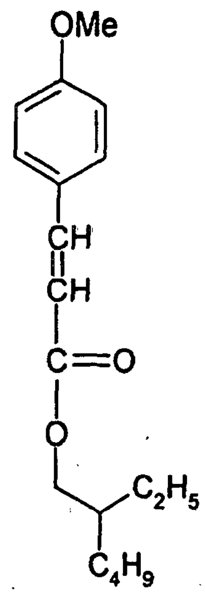

Parmi les composés (B) mentionnés ci-dessus, on préfère particulièrement, selon la présente invention, mettre en oeuvre le salicylate d'homomenthyle, connu aussi sous le nom d'homosalate, tel que le produit disponible commercialement sous la dénomination de "KEMESTER HMS" par la Société Witco. Il répond à la formule suivante :

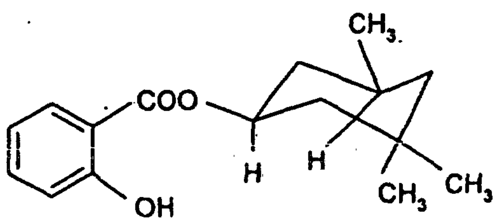

Parmi les les composés (B) mentionnés ci-dessus, on préfère encore plus particulièrement, selon la présente invention, mettre en oeuvre le salicylate d'octyle, vendu notamment sous la dénomination commerciale de "UVINUL O-18" par la Société BASF, et répondant à la formule suivante :

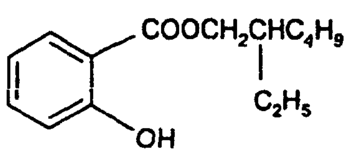

Parmi les composés (B) utilisables selon la présente invention, on peut citer également les composés ou les mélanges de composés répondant à la formule (I) suivante :

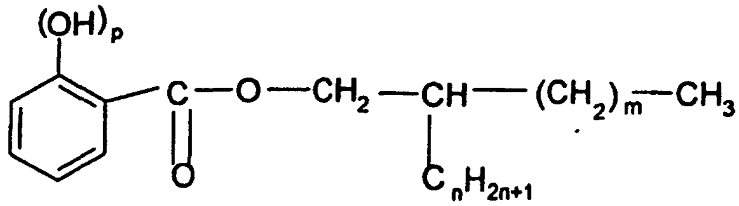

(I)

dans laquelle m vaut 5, 7 ou 9 ; n vaut 4, 6 ou 8 ; p vaut 0 ou 1 ; sous réserve que lorsque p vaut 0 alors m vaut 5 ou 7 et n vaut 4 ou 6.

Ces composés sont décrits et préparés dans le brevet US 5,783,173. Parmi ces composés de formule (I), on peut citer le benzoate de 2-butyloctyle, le benzoate de 2-hexyldécyle, l'hydroxybenzoate de 2-butyloctyle ou leurs mélanges et plus particulièrement le mélange benzoate de 2-butyloctyle/benzoate de 2-hexyldécyle tels que le produit commercial « ALLSTAR AB » vendu par la Société CP HALL ou l'hydroxybenzoate de 2-butyloctyle tel que le produit commercial « HALLBRITE BHB » vendu par la société CP HALL.

Le ou les composés (A) et/ou le ou les composés (B) de l'invention sont présents à des teneurs allant de 0,1 à 20 % en poids et de préférence allant de 0,2 à 15% par rapport au poids total de la composition.

Selon une caractéristique essentielle des compositions selon l'invention, ces composés doivent être utilisés, seuls ou en mélanges, en une quantité telle qu'elle soit suffisante pour solubiliser à elle seule la totalité, ou substantiellement la totalité du ou des filtres du type dérivé silicié à fonction benzotriazole dans la composition. Cette quantité minimale en solvant(s) destinée à assurer une dissolution complète et stable du ou des filtres solides de l'invention peut être classiquement déterminée à partir de l'étude des paramètres de solubilité desdits filtres dans ces solvants.

A titre d'exemple, il a été constaté qu'à température ambiante, la silicone benzotriazole correspondant au composé (a) tel que décrit précédemment est soluble à raison de 50 % en poids dans le p-méthoxycinnamate de 2-éthylhexyle (filtre solvant du type cinnamate) et à raison de 60% en poids dans le salicylate d'octyle (filtre solvant du type salicylate).

D'une manière générale, on notera que les concentrations en composés dérivés siliciés à fonction benzotriazole et en composés cinnammates et/ou salicylates, sont choisies de manière telle que l'indice de protection solaire de la composition finale soit de préférence d'au moins 2.

Selon une caractéristique particulièrement avantageuse des compositions conformes à l'invention, ces dernières ne contiennent de préférence aucun, ou substantiellement aucun, solubilisant pour les composés dérivés siliciés à foncbenzotriazole autre que les dérivés de l'acide cinnamique ou les dérivés de l'acide salicylique précédemment définis. Selon l'invention, on considère qu'un composé donné ne possède pas de propriétés solubilisantes vis à vis d'un autre composé donné lorsque ce dernier composé présente une solubilité inférieure à 1 % en poids environ dans ce premier composé.

En outre, toujours selon un mode préféré de réalisation de la présente invention, le support cosmétiquement acceptable dans lequel se trouvent contenus les différents systèmes filtrants, est une émulsion de type huile-dans-eau.

Les compositions cosmétiques antisolaires selon l'invention peuvent bien entendu contenir un ou plusieurs filtres solaires complémentaires actifs dans l'UVA et/ou l'UVB (absorbeurs), hydrophiles ou lipophiles autres, bien sûr, que tous les filtres lipophiles mentionnés ci-avant. Ces filtres complémentaires peuvent être notamment choisis parmi les dérivés du camphre, les dérivés de triazine, les dérivés de la benzophénone, les dérivés du dibenzoylméthane, les dérivés de β,β-diphénylacrylate, les dérivés de l'acide p-aminobenzoïque, les polymères filtres et silicones filtres décrits dans la demande WO-93/04665. D'autres exemples de filtres organiques sont donnés dans la demande de brevet EP-A 0 487 404.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A-0518772 et EP-A-0518773.

Les compositions de l'invention peuvent comprendre en outre des adjuvants cosmétiques classiques notamment choisis parmi les corps gras, les solvants organiques, ies épaississants, les adoucissants, les antioxydants, les opacifiants, les stabilisants, les émollients, les hydroxyacides, les agents anti-mousse, les agents hydratants, les vitamines, les parfums, les conservateurs, les tensioactifs, les charges, les séquestrants, les propulseurs, les agents

alcalinisants ou acidifiants, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-$\alpha$-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les homopolymères d'acide acrylique réticulés, les gommes de guar et celluloses modifiées ou non telles que la gomme de guar hydroxypropylée, la méthylhydroxyéthylcellulose, l'hydroxypropylméthyl cellulose ou encore l'hydroxyéthylcellulose.

Bien entendu, l'homme de l'art veillera à choisir ce ou ces éventuels composés complémentaires et/ou leurs quantités de manière telle que les propriétés avantageuses, en particulier les facteurs de protection solaire obtenus, attachées intrinsèquement à l'association de filtres conforme à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion la phase aqueuse de celle-ci peut comprendre une dispersion vésiculaire non ionique préparée selon des procédés connus (Bangham, Standish and Watkins. J. Mol. Biol. 13, 238 (1965), FR 2315991 et FR 2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique et peut constituer par exemple une composition à rincer à appliquer avant ou après shampooing, avant ou après coloration ou décoloration, avant pendant ou après permanente ou défrisage, une lotion ou un gel coiffants où traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Comme indiqué en début de description, un autre objet de la présente invention réside dans un procédé de traitement cosmétique de la peau ou des cheveux destiné à les protéger contre les effets des rayons UV consistant à appliquer sur ceux-ci une quantité efficace d'une composition cosmétique telle que définie ci-dessus.

Des exemples concrets, mais nullement limitatifs, illustrant l'invention, vont maintenant être donnés.

**Exemple 1 : Crème antisolaire (émulsion huile/eau)**

- Silicone benzotriazole correspondant au composé (a) 5g
- Cinnamate de 2-éthylhexyle (Parsol MCX ) 10 g
- Emulsionnant (Arlacel 165) 2g

- Acide stéarique 2,5g
- Conservateur QS
- Alcool stéarylique 0,5g
- Triéthanolamine 0,5g

- Hydratants 8g
- Conservateur QS
- Sequestrant 0,1g

- Polymère épaississant acrylique (Pemulen TR1) 0.22g
- Polydiméthylsiloxane (DC245 Fluid) 2g

- Triéthanolamine 0,22g

- Eau purifiée QS 100g

On réalise l'émulsion ci-dessus en dissolvant les filtres dans la phase grasse, puis en ajoutant les émulsionnants dans cette phase grasse portée aux environs de 80°C, et enfin en additionnant sous agitation rapide l'eau préalablement chauffée à cette même température.

**EXEMPLE 2**

- Silicone benzotriazole correspondant au composé (a) 6 g
- Salicylate d'octyle 10 g
- Emulsionnant (Arlacel 165) 2g
- Acide stéarique 2,5g
- Conservateur QS
- Alcool stéarylique 0,5g
- Triéthanolamine 0,5g

- Hydratants 8g
- Conservateur QS
- Sequestrant 0.1g

- Polymère épaississant acrylique (Pemulen TR1) 0,22g
- Polydiméthylsiloxane (DC245 Fluid) 2g

- Triéthanolamine 0,22g

- Eau purifiée QS 100g

On réalise l'émulsion ci-dessus en dissolvant les filtres dans la phase grasse, puis en ajoutant les émulsionnants dans cette phase grasse portée aux environs de 80°C, et enfin en additionnant sous agitation rapide l'eau préalablement chauffée à cette même température.

**Revendications**

1. Composition cosmétique à usage topique, en particulier pour la photoprotection de la peau et/ou des cheveux, **caractérisées par le fait qu'**elle comprend dans un support cosmétiquement acceptable,

    (i) un système filtrant solubilisé constitué d'au moins un dérivé silicié à fonction benzotriazole répondant à l'une des formules (5) ou (6) suivantes :

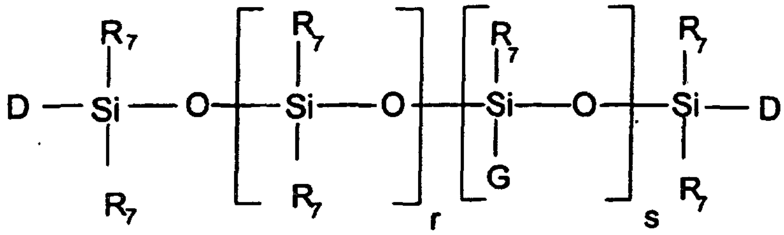

(5)

ou

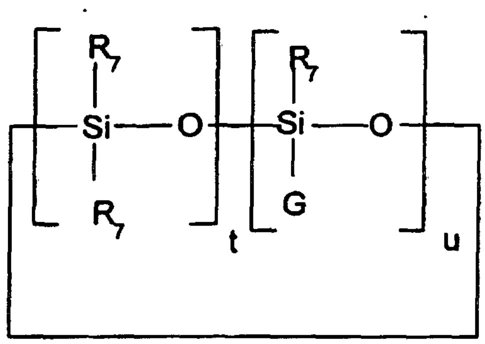

(6)

dans lesquelles :

- $R_7$, identiques ou différents, sont choisis parmi les radicaux alkyles en $C_1$-$C_{10}$, phényle, trifluoro-3,3,3 propyle et triméthylsilyloxy, au moins 80% en nombre des radicaux $R_7$ étant méthyle,
- D, identiques ou différents sont choisis parmi les radicaux $R_7$ et le radical G,
- r est un nombre entier compris entre 0 et 50 inclusivement, et s est un nombre entier compris entre 0 et 20 inclusivement, et si s = 0, au moins l'un des deux symboles D désigne G,
- u est un nombre entier compris entre 1 et 6 inclusivement, et t est un nombre entier compris entre 0 et 10 inclusivement, étant entendu que t + u est égal ou supérieur à 3,
- et le symbole G répond à la formule (2)

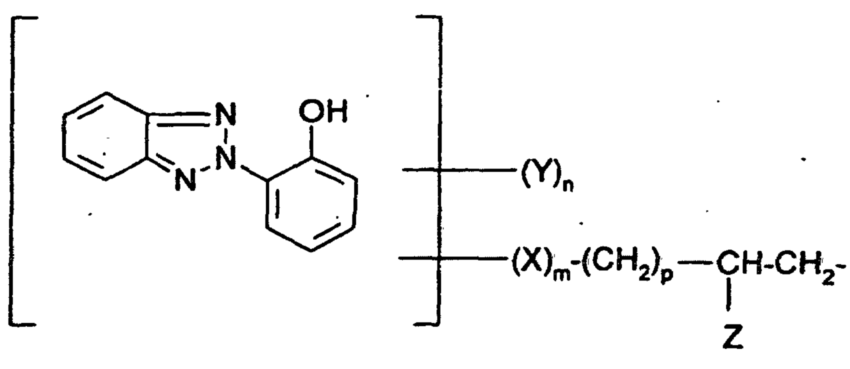

(2)

dans laquelle :

- Y, identiques ou différents, sont choisis parmi les radicaux alkyles en $C_1$-$C_8$, les halogènes et les radicaux alkoxy en $C_1$-$C_4$ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z représente l'hydrogène ou un radical alkyle en $C_1$-$C_4$,
- n est un nombre entier compris entre 0 et 3 inclusivement,

- m est 0 ou 1,
- p représente un nombre entier compris entre 1 et 10, inclusivement.

(ii) un système filtrant solubilisant constitué :

- d'au moins un composé (A) choisi dans le groupe formé par le 4-méthoxy cinnamate d'isopentyle, le 4-méthoxy cinnamate de 2-éthylhexyle, le diisopropyl cinnamate de méthyle, le 4-méthoxy cinnamate d'isoamyle, le 4-méthoxy cinnamate de diéthanolamine et/ou
- d'au moins un composé (B) choisi dans le groupe constitué par le salicylate d'homomenthyle, le salicylate de 2-éthylhexyle, le salicylate de triéthanolamine, le salicylate de 4-isopropylbenzyle et les composés ou les mélanges de composés répondant à la formule (I) suivante :

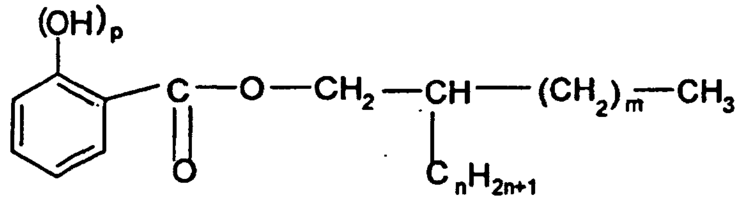

**(I)**

dans laquelle m vaut 5, 7 ou 9 ; n vaut 4, 6 ou 8 ; p vaut 0 ou 1 ; sous réserve que lorsque p vaut 0 alors m vaut 5 ou 7 et n vaut 4 ou 6 ;

ledit système filtrant solubilisant étant présent en une quantité suffisante pour solubiliser à lui seul l'ensemble dudit système filtrant solubilisé.

**2.** Composition selon la revendication 1, **caractérisée par le fait que** dérivé silicié à fonction benzotriazole répond à la formule (7) suivante :

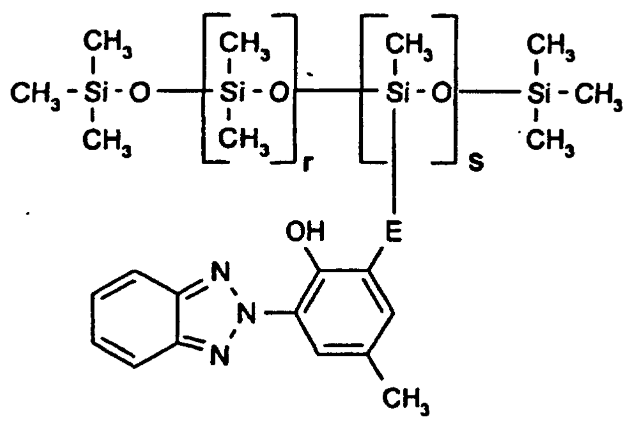

**(7)**

avec

$$0 \leq r \leq 10,$$

$$1 \leq s \leq 10,$$

et où E représente le radical divalent :

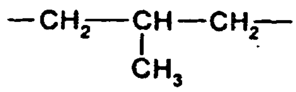

3. Composition selon la revendication 2, **caractérisée par le fait que** le dérivé silicié à fonction benzotriazole répond à la formule suivante :

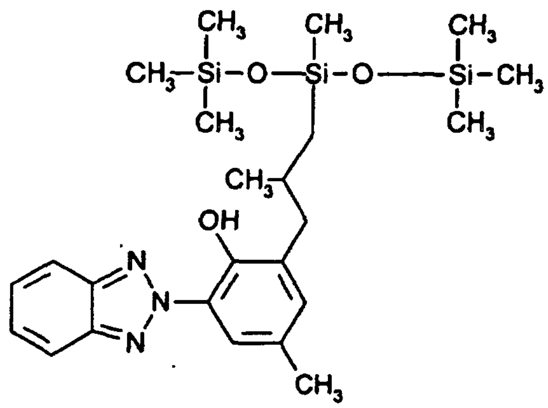

4. Composition selon l'une quelconque des revendications 1 à 3, **caractérisée par le fait que** les dérivés siliciés à fonction benzotriazole sont présents à une teneur allant de 0, 1 % à 20 %, de préférence de 0,2 % à 15 % en poids, par rapport au poids total de la composition.

5. Composition selon l'une quelconque des revendications 1 à 4, où le composé (A) est le p- méthoxycinnamate de 2-éthylhexyle répondant à la formule développée suivante :

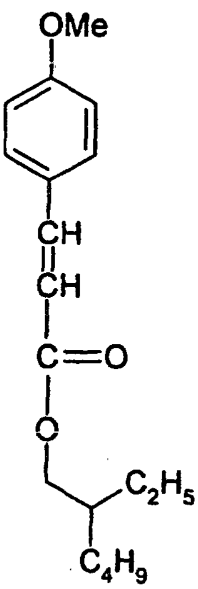

6. Composition selon l'une quelconque des revendications 1 à 4, où le composé (B) est le salicylate d'homomenthyle de formule suivante :

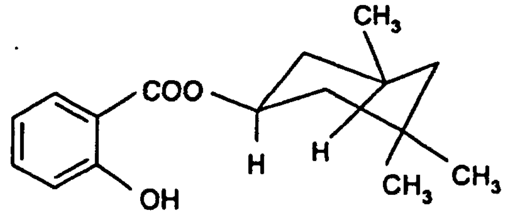

**7.** Composition selon l'une quelconque des revendications 1 à 4, où le composé (B) est le le salicylate d'octyle répondant à la formule suivante :

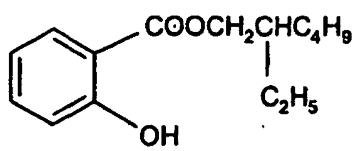

**8.** Composition selon l'une quelconque des revendications 1 à 4, où le ou les composés de formule (I) sont choisis parmi le benzoate de 2-butyloctyle, le benzoate de 2-hexyldécyle, l'hydroxybenzoate de 2-butyloctyle ou leurs mélanges.

**9.** Composition selon l'une quelconque des revendications 1 à 8, où le système filtrant sont présents à des teneurs allant de 0,1 à 20 % en poids et de préférence allant de 0,2 à 15% par rapport au poids total de la composition.

**10.** Composition selon l'une quelconque des revendications 1 à 9, **caractérisée par le fait qu'**elle ne contient aucun, ou substantiellement aucun autre système solubilisant le système filtrant solubilisé que le système filtrant solubilisant tel que défini dans les revendications précédentes.

**11.** Composition selon l'une quelconque des revendications 1 à 10, **caractérisée par le fait que** ledit support cosmétiquement acceptable se présente sous la forme d'une émulsion de type huile-dans-eau.

**12.** Composition selon l'une quelconque des revendications 1 à 11, **caractérisée par le fait qu'**elles comprend en outre un ou plusieurs filtres organiques complémentaires actifs dans l'UV-A et/ou UV-B, hydrophiles ou lipophiles.

**13.** Composition selon la revendication 12, **caractérisée par le fait que** lesdits filtres organiques complémentaires sont choisis parmi les dérivés du camphre, les dérivés de triazine, les dérivés de la benzophénone, les dérivés du dibenzoylméthane, les dérivés de β,β-diphénylacrylate, les dérivés de l'acide p-aminobenzoïque, les polymères filtres et les silicones filtres autres que les dérivés siliciés à fonction benzotriazole.

**14.** Composition selon l'une quelconque des revendications 1 à 13, **caractérisée par le fait qu'**elles comprend en outre, à titre d'agents photoprotecteurs complémentaires, des pigments ou nanopigments d'oxydes métalliques, enrobés ou non, capables de bloquer physiquement, par diffusion et/ou réflection, le rayonnement UV.

**15.** Composition selon la revendication 14, **caractérisée par le fait que** lesdits pigments ou nanopigments sont choisis parmi les oxydes de titane, de zinc, de fer, de zirconium, de cérium et leurs mélanges, enrobés ou non.

**16.** Composition selon l'une quelconque des revendications 1 à 15, **caractérisée par le fait qu'**elles comprend en outre au moins un agent de bronzage et/ou de brunissage artificiel de la peau.

**17.** Composition selon l'une quelconque des revendications 1 à 16, **caractérisée par le fait qu'**elle comprend en outre au moins un adjuvant choisi parmi les corps gras, les solvants organiques, les épaississants, les adoucissants, les antioxydants, les opacifiants, les stabilisants, les émollients, les hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants.

**18.** Composition selon l'une quelconque des revendications 1 à 17, **caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une poudre, d'un bâtonnet solide, d'une mousse ou d'un spray.

**19.** Composition selon l'une quelconque des revendications 1 à 18, **caractérisée par le fait qu'**il s'agit d'une composition de maquillage des cils, des sourcils ou de la peau et qu'elle se présente sous forme solide ou pâteuse, anhydre ou aqueuse, d'une émulsion, d'une suspension ou d'une dispersion.

**20.** Composition selon l'une quelconque des revendications 1 à 18, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

**21.** Utilisation de la composition définie à l'une quelconque des revendications 1 à 20 pour la fabrication de, compositions cosmétiques pour la protection de la peau et/ou des cheveux contre le rayonnement ultraviolet, en particulier le rayonnement solaire.

**22.** Utilisation pour la fabrication d'une composition cosmétique pour la protection de la peau et/ou des cheveux contre les radiations UV, d'au moins un composé (A) et/ou d'au moins composé (B) tels que définis dans les revendications 1 à 8 comme système filtrant solubilisant permettant dans une quantité suffisante de solubiliser à lui seul au moins un filtre UV solide du type dérivé silicié à fonction benzatriazole tel que définis dans les revendications 1 à 3 présent dans ladite composition.

**Claims**

**1.** Cosmetic composition for topical use, in particular for the photoprotection of the skin and/or hair, **characterized in that** it comprises, in a cosmetically acceptable vehicle,

(i) a dissolved screening system composed of at least one silicon derivative with a benzotriazole functional group corresponding to one of the following formulae (5) or (6):

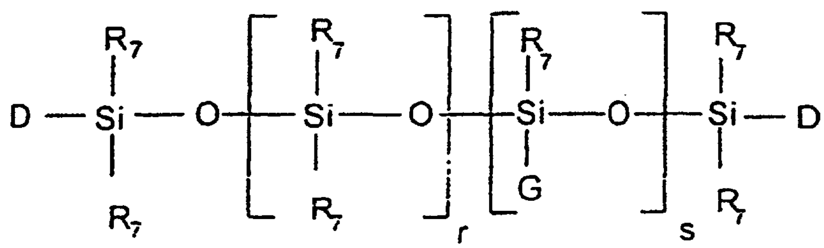

(5)

or

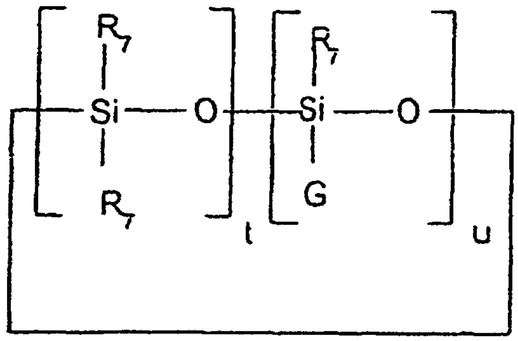

(6)

in which

- $R_7$, which are identical or different, are chosen from $C_1$-$C_{10}$ alkyl, phenyl, 3,3,3-trifluoropropyl and trimethylsilyloxy radicals, at least 80% by number of the $R_7$ radicals being methyl,
- D, which are identical or different, are chosen from $R_7$ radicals and the G radical,
- r is an integer between 0 and 50 inclusive and s is an integer between 0 and 20 inclusive and, if s = 0, at least one of the two D symbols denotes G,
- u is an integer between 1 and 6 inclusive and t is an integer between 0 and 10 inclusive, it being understood that t + u is equal to or greater than 3,
- and the G symbol corresponds to the formula (2)

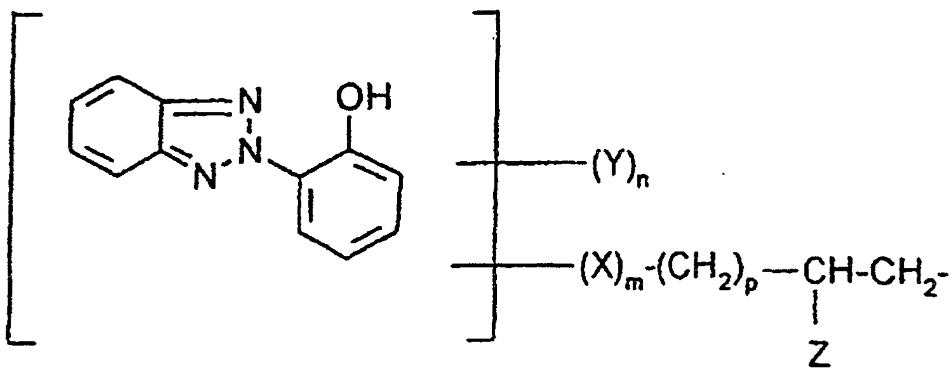

(2)

in which:

- Y, which are identical or different, are chosen from $C_1$-$C_8$ alkyl radicals, halogens and $C_1$-$C_4$ alkoxy radicals, it being understood that, in the latter case, two adjacent Y radicals on the same aromatic nucleus can together form an alkylidenedioxy group in which the alkylidene group comprises from 1 to 2 carbon atoms,
- X represents O or NH,
- Z represents hydrogen or a $C_1$-$C_4$ alkyl radical,
- n is an integer between 0 and 3 inclusive,
- m is 0 or 1,
- p represents an integer between 1 and 10 inclusive;

(ii) a solubilizing screening system composed:

- of at least one compound (A) chosen from the group formed by isopentyl 4-methoxycinnamate, 2-ethylhexyl 4-methoxycinnamate, methyl diisopropylcinnamate, isoamyl 4-methoxycinnamate or diethanolamine 4-methoxycinnamate and/or
- of at least one compound (B) chosen from the group composed of homomenthyl salicylate, 2-ethylhexyl

salicylate, triethanolamine salicylate or 4-isopropylbenzyl salicylate and compounds or mixtures of compounds corresponding to the following formula (I)

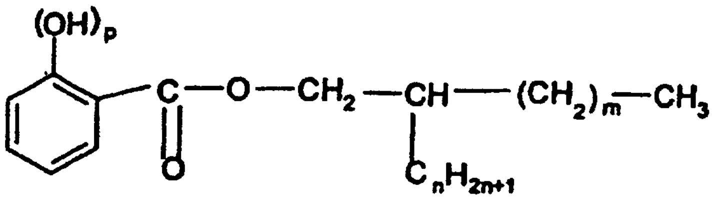

(I)

in which m has the value 5, 7 or 9, n has the value 4, 6 or 8 and p has the value 0 or 1, with the proviso that, when p has the value 0, then m has the value 5 or 7 and n has the value 4 or 6;

the said solubilizing screening system being present in an amount sufficient to dissolve, by itself alone, the whole of the said dissolved screening system.

**2.** Composition according to Claim 1, **characterized in that** the silicon derivative with a benzotriazole functional group corresponds to the following formula (7) :

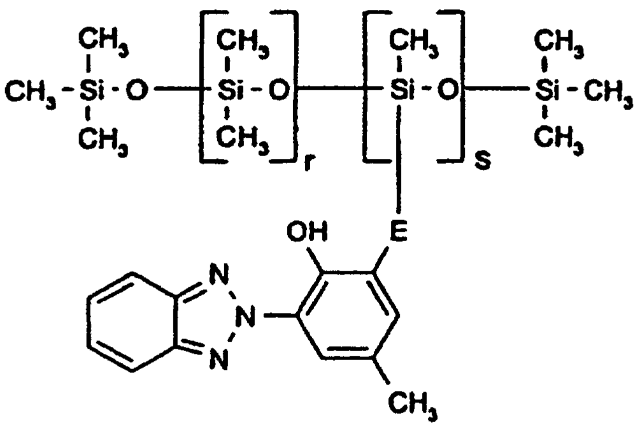

(7)

with

$$0 \leq r \leq 10,$$

$$1 \leq s \leq 10,$$

and where E represents the divalent radical:

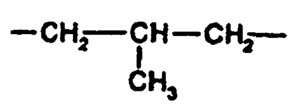

**3.** Composition according to Claim 2, **characterized in that** the silicon derivative with a benzotriazole functional group corresponds to the following formula:

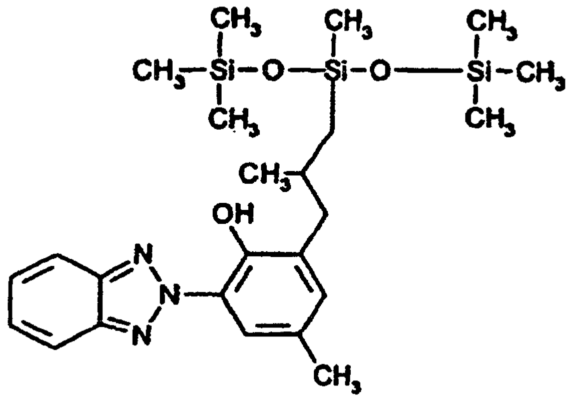

4. Composition according to any one of Claims 1 to 3, **characterized in that** the silicon derivatives with a benzotriazole functional group are present at a content ranging from 0.1% to 20%, preferably from 0.2% to 15%, by weight with respect to the total weight of the composition.

5. Composition according to any one of Claims 1 to 4, where the compound (A) is 2-ethylhexyl p-methoxycinnamate corresponding to the following expanded formula:

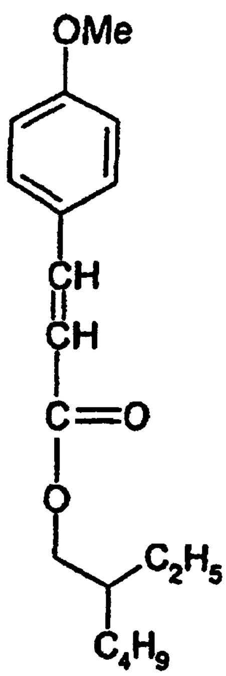

6. Composition according to any one of Claims 1 to 4, where the compound (B) is homomenthyl salicylate of following formula:

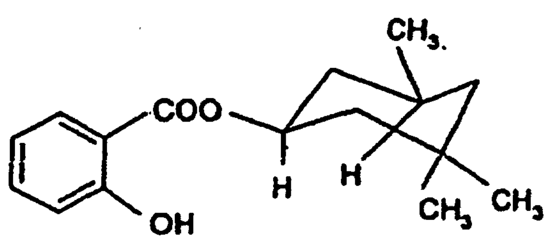

**7.** Composition according to any one of Claims 1 to 4, where the compound (B) is octyl salicylate corresponding to the following formula:

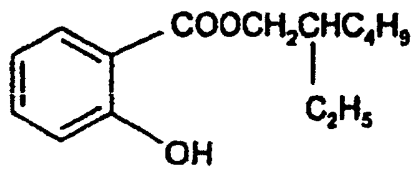

**8.** Composition according to any one of Claims 1 to 4, where the compound or compounds of formula (I) are chosen from 2-butyloctyl benzoate, 2-hexyldecyl benzoate, 2-butyloctyl hydroxybenzoate or their mixtures.

**9.** Composition according to any one of Claims 1 to 8, where the screening system is present at contents ranging from 0.1 to 20% by weight and preferably ranging from 0.2 to 15% with respect to the total weight of the composition.

**10.** Composition according to any one of Claims 1 to 9, **characterized in that** it comprises no, or substantially no, other system solubilizing the dissolved screening system than the solubilizing screening system as defined in the preceding claims.

**11.** Composition according to any one of Claims 1 to 10, **characterized in that** the said cosmetically acceptable vehicle is provided in the form of an emulsion of oil-in-water type.

**12.** Composition according to any one of Claims 1 to 11, **characterized in that** it additionally comprises one or more additional, hydrophilic or lipophilic, organic screening agents which are active in the UV-A and/or UV-B.

**13.** Composition according to Claim 12, **characterized in that** the said additional organic screening agents are chosen from camphor derivatives, triazine derivatives, benzophenone derivatives, dibenzoylmethane derivatives, β,β-diphenylacrylate derivatives, p-aminobenzoic acid derivatives or polymer screening agents and silicone screening agents, other than silicon derivatives with a benzotriazole functional group.

**14.** Composition according to any one of Claims 1 to 13, **characterized in that** it additionally comprises, as additional photoprotective agents, coated or non-coated metal oxide pigments or nanopigments capable of physically blocking, by scattering and/or reflection, the UV radiation.

**15.** Composition according to Claim 14, **characterized in that** the said pigments or nanopigments are chosen from coated or non-coated titanium oxide, zinc oxide, iron oxide, zirconium oxide, cerium oxide and their mixtures.

**16.** Composition according to any one of Claims 1 to 15, **characterized in that** it additionally comprises at least one agent for the artificial bronzing and/or tanning of the skin.

**17.** Composition according to any one of Claims 1 to 16, **characterized in that** it additionally comprises at least one adjuvant chosen from fatty substances, organic solvents, thickeners, softeners, antioxidants, opacifying agents, stabilizers, emollients, hydroxy acids, antifoaming agents, moisturizing agents, vitamins, fragrances, preservatives, surfactants, fillers, sequestering agents, polymers, propellants, basifying or acidifying agents, or dyes.

**18.** Composition according to any one of Claims 1 to 17, **characterized in that** it is a protective composition for the human skin or an antisun composition and **in that** it is provided in the form of a nonionic vesicular dispersion, of an emulsion, in particular of an emulsion of oil-in-water type, of a cream, of a milk, of a gel, of a cream gel, of a suspension, of a dispersion, of a powder, of a solid stick, of a foam or of a spray.

**19.** Composition according to any one of Claims 1 to 18, **characterized in that** it is a make-up composition for the eyelashes, eyebrows or skin and **in that** it is provided in the form, anhydrous or aqueous, solid or pasty, of an emulsion, of a suspension or of a dispersion.

**20.** Composition according to any one of Claims 1 to 18, **characterized in that** it is a composition intended for the

protection of the hair against ultraviolet radiation and **in that** it is provided in the form of a shampoo, of a lotion, of a gel, of an emulsion or of a nonionic vesicular dispersion.

**21.** Use of the composition defined in any one of Claims 1 to 20 in the manufacture of cosmetic compositions for the protection of the skin and/or hair against ultraviolet radiation, in particular solar radiation.

**22.** Use, in the manufacture of a cosmetic composition for the protection of the skin and/or hair against UV radiation, of at least one compound (A) and/or of at least one compound (B) as defined in Claims 1 to 8 as solubilizing screening system making it possible, in a sufficient amount, to dissolve, by itself alone, at least one solid UV screening agent of the silicon derivative with a benzotriazole functional group type as defined in Claims 1 to 3 present in the said composition.

**Patentansprüche**

**1.** Kosmetische Zusammensetzung zur topischen Anwendung, insbesondere zum Lichtschutz der Haut und/oder der Haare,
**dadurch gekennzeichnet, dass** sie in einem kosmetisch akzeptablen Träger enthält:

(i) ein solubilisiertes Filtersystem, das aus mindestens einem siliciumhaltigen Derivat mit Benzotriazolgruppe besteht, das einer der folgenden Formeln (5) oder (6) entspricht:

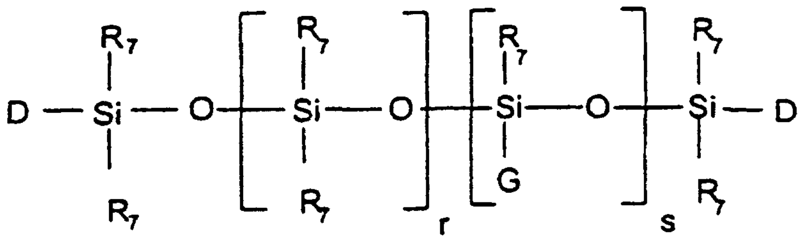

(5)

oder

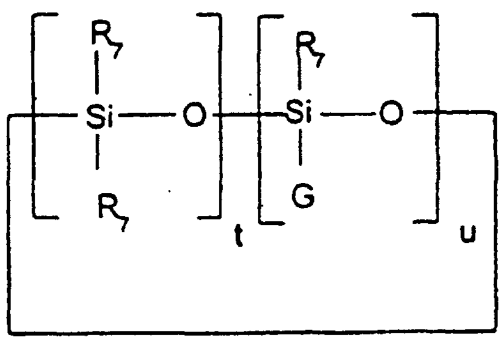

(6),

worin:

- die Gruppen $R_7$, die identisch oder voneinander verschieden sind, unter den $C_{1-10}$-Alkylgruppen, Phenyl, 3,3,3-Trifluorpropyl und Trimethylsilyloxy ausgewählt sind, wobei mindestens 80 % der Anzahl der Gruppen $R_7$ Methyl bedeutet,
- die Gruppen D, die identisch oder voneinander verschieden sind, unter den Gruppen $R_7$ und der Gruppe G ausgewählt sind,
- r 0 oder eine ganze Zahl im Bereich von 1 bis 50 und s 0 oder eine ganze Zahl im Bereich von 1 bis 20

bedeutet, wobei die Bereichsgrenzen jeweils eingeschlossen sind und mindestens eine der beiden Gruppen D G bedeutet, wenn s 0 ist,

- u eine ganze Zahl im Bereich von 1 bis 6 und t 0 oder eine ganze Zahl im Bereich von 1 bis 10 ist, wobei die Bereichsgrenzen jeweils eingeschlossen sind, mit der Maßgabe, dass t + u 3 bedeutet oder darüber liegt, und
- die Gruppe G der Formel (2) entspricht:

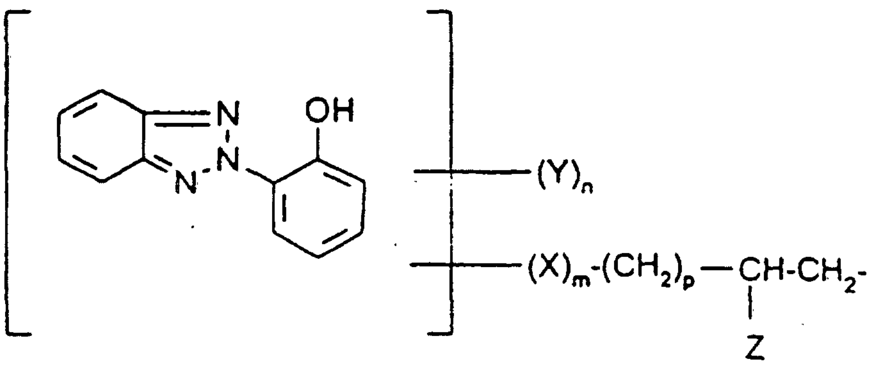

(2),

worin:

- die Gruppen Y, die identisch oder voneinander verschieden sind, unter den $C_{1-8}$-Alkylgruppen, Halogenen und $C_{1-4}$-Alkoxygruppen ausgewählt sind, wobei im letzten Fall zwei an dem gleichen aromatischen Ring aneinander angrenzende Gruppen Y auch gemeinsam eine Alkylidendioxygruppe bilden können, worin die Alkylidengruppe 1 bis 2 Kohlenstoffatome aufweist,
- X O oder NH bedeutet,
- Z Wasserstoff oder eine $C_1$-$_4$-Alkylgruppe bedeutet,
- n 0 oder eine ganze Zahl im Bereich von 1 bis 3 ist, wobei die Bereichsgrenzen eingeschlossen sind,
- m 0 oder 1 ist, und
- p eine ganze Zahl im Bereich von 1 bis 10 bedeutet, wobei die Bereichsgrenzen eingeschlossen sind; und

(ii) ein solubilisierendes Filtersystem, bestehend aus:

- mindestens einer Verbindung (A), die unter Isopentyl-4-methoxy-cinnamat, 2-Ethylhexyl-4-methoxy-cinnamat, Methyldiisopropyl-cinnamat, Isoamyl-4-methoxy-cinnamat und Diethanolamin-4-methoxy-cinnamat ausgewählt ist, und/oder
- mindestens einer Verbindung (B), die unter Homomenthylsalicylat, 2-Ethylhexyl-salicylat, Triethanolaminsalicylat, 4-Isopropylbenzyl-salicylat und den Verbindungen oder Gemischen von Verbindungen der folgenden Formel (I) ausgewählt sind:

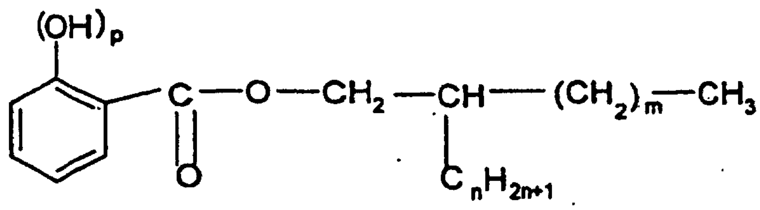

(I),

worin m 5, 7 oder 9; n 4, 6 oder 8; und p 0 oder 1 bedeutet, mit der Maßgabe, dass m 5 oder 7 und n 4 oder 6 bedeutet, wenn p Null ist,

wobei das solubilisierende Filtersystem in einer Menge enthalten ist, die alleine ausreicht, das gesamte solubilisierte Filtersystem zu solubilisieren.

2. Zusammensetzung nach Anspruch 1, **dadurch gekennzeichnet, dass** das siliciumhaltige Derivat mit Benzotriazolgruppe der folgenden Formel (7) entspricht:

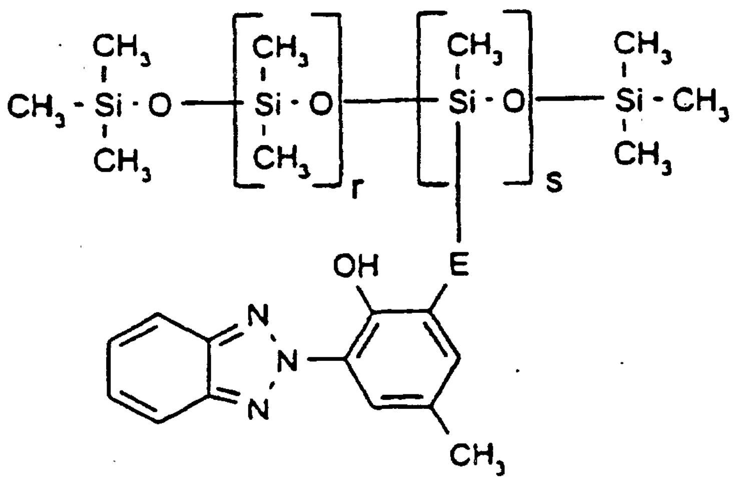

(7)

worin bedeuten:

$$0 \leq r \leq 10,$$

$$1 \leq s \leq 10,$$

und E die zweiwertige Gruppe:

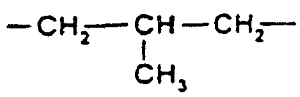

3. Zusammensetzung nach Anspruch 2, **dadurch gekennzeichnet, dass** das siliciumhaltigen Derivat mit Benzotriazolgruppe der folgenden Formel entspricht:

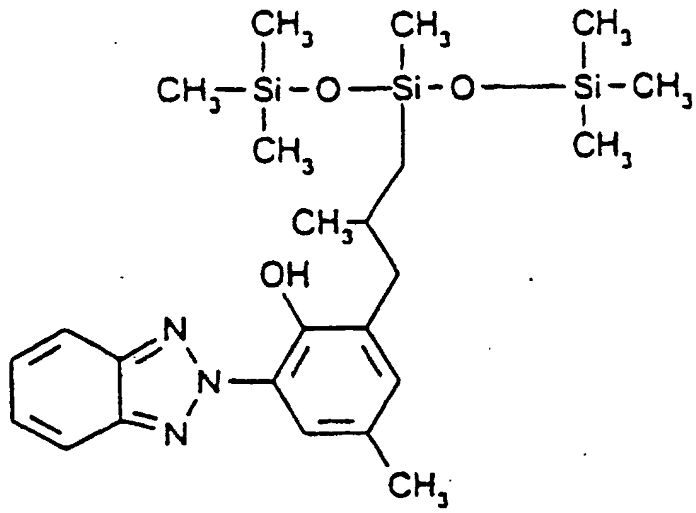

4. Zusammensetzung nach einem der Ansprüche 1 bis 3, **dadurch gekennzeichnet, dass** das siliciumhaltige Derivat mit Benzotriazolgruppe in einem Mengenanteil von 0,1 bis 20 Gew.-% und vorzugsweise 0,2 bis 15 Gew.-%, bezogen auf das Gesamtgewicht der Zusammensetzung, enthalten ist.

5. Zusammensetzung nach einem der Ansprüche 1 bis 4, wobei die Verbindung (A) das 2-Ethylhexyl-p-methoxycinnamat der folgenden Strukturformel ist:

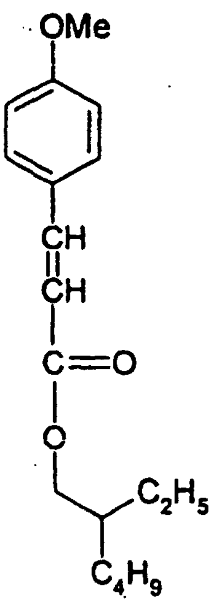

6. Zusammensetzung nach einem der Ansprüche 1 bis 4, wobei die Verbindung (B) das Homomenthylsalicylat der folgenden Formel ist:

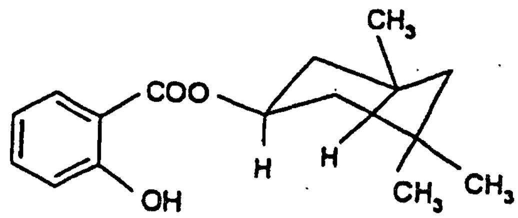

7. Zusammensetzung nach einem der Ansprüche 1 bis 4, wobei die Verbindung (B) das Octylsalicylat der folgenden Formel ist:

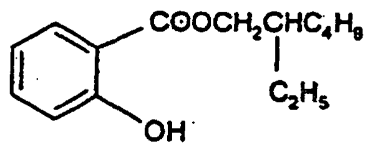

8. Zusammensetzung nach einem der Ansprüche 1 bis 4, wobei die Verbindung(en) der Formel (I) unter 2-Butyloctylbenzoat, 2-Hexyldecylbenzoat, 2-Butyloctylhydroxybenzoat oder deren Gemischen ausgewählt ist.

9. Zusammensetzung nach einem der Ansprüche 1 bis 8, wobei das Filtersystem in einer Menge von 0,1 bis 20 Gew.-% und vorzugsweise 0,2 bis 15 %, bezogen auf das Gesamtgewicht der Zusammensetzung, vorliegt.

10. Zusammensetzung nach einem der Ansprüche 1 bis 9, **dadurch gekennzeichnet, dass** sie kein oder im Wesentlichen kein anderes System enthält, das das solubilisierte Filtersystem solubilisiert und von dem in den vorhergehenden Ansprüchen definierten solubilisierenden Filtersystem verschieden ist.

11. Zusammensetzung nach einem der Ansprüche 1 bis 10, **dadurch gekennzeichnet, dass** der kosmetisch akzeptable Träger in Form einer Öl-in-Wasser-Emulsion vorliegt.

12. Zusammensetzung nach einem der Ansprüche 1 bis 11, **dadurch gekennzeichnet, dass** sie ferner ein oder mehrere ergänzende hydrophile oder lipophile organische Filter, die im UV-A-Bereich und/oder UV-B-Bereich wirksam sind, enthält.

13. Zusammensetzung nach Anspruch 12, **dadurch gekennzeichnet, dass** die ergänzenden organischen Filter unter den Campherderivaten, Triazinderivaten, Benzophenonderivaten, Dibenzoylmethanderivaten, β,β-Diphenylacrylatderivaten, p-Aminobenzoesäurederivaten, polymeren Filtern und Siliconfiltern, die von den beschriebenen siliciumhaltigen Derivaten mit Benzotriazolfunktion verschieden sind, ausgewählt werden.

14. Zusammensetzung nach einem der Ansprüche 1 bis 13, **dadurch gekennzeichnet, dass** sie als ergänzende Lichtschutzmittel ferner gegebenenfalls umhüllte Pigmente oder Nanopigmente von Metalloxiden enthält, die die UV-Strahlung physikalisch durch Streuung und/oder Reflexion zu sperren vermögen.

15. Zusammensetzung nach Anspruch 14, **dadurch gekennzeichnet, dass** die Pigmente und Nanopigmente unter gegebenenfalls umhüllten Oxiden von Titan, Zink, Eisen, Zirconium, Cer und deren Gemischen ausgewählt sind.

16. Zusammensetzung nach einem der Ansprüche 1 bis 15, **dadurch gekennzeichnet, dass** sie außerdem mindestens ein Bräunungsmittel und/oder Mittel zur künstlichen Braunfärbung der Haut enthält.

17. Zusammensetzung nach einem der Ansprüche 1 bis 16, **dadurch gekennzeichnet, dass** sie ferner mindestens einen Zusatzstoff enthält, der unter den Fettsubstanzen, organischen Lösungsmitteln, Verdickungsmitteln, beruhigenden Stoffen, Antioxidantien, Trübungsmitteln, Stabilisatoren, Emollientien, Hydroxysäuren, Schaumverhütungsmitteln, Hydratisierungsmitteln, Vitaminen, Parfums, Konservierungsmitteln, grenzflächenaktiven Stoffen, Füllstoffen, Maskierungsmitteln, Polymeren, Treibmitteln, Alkalisierungsmitteln, Ansäuerungsmitteln und Farbmitteln ausgewählt ist.

18. Zusammensetzung nach einem der Ansprüche 1 bis 17, **dadurch gekennzeichnet, dass** es sich um Zusammensetzungen, die die menschliche Epidermis schützen, oder Sonnenschutzzusammensetzungen handelt und diese als nichtionische Vesikeldispersionen, Emulsionen, insbesondere Öl-in-Wasser-Emulsionen, Cremes, Milche, Gele, Gelcremes, Suspensionen, Dispersionen, Puder, feste Stifte, Schäume und Sprays vorliegen.

19. Zusammensetzung nach einem der Ansprüche 1 bis 18, **dadurch gekennzeichnet, dass** es sich um Zusammensetzungen zum Schminken der Wimpern, Augenbrauen oder der Haut handelt und dass sie in fester oder pastöser, wasserfreier oder wässriger Form, als Emulsionen, Suspensionen oder Dispersionen vorliegen.

20. Zusammensetzung nach einem der Ansprüche 1 bis 18, **dadurch gekennzeichnet, dass** es sich um Zusammensetzungen zum Schutz der Haare gegen UV-Strahlung handelt und dass sie als Haarwaschmittel, Lotionen, Gele, Emulsionen oder nichtionische Vesikeldispersionen vorliegen.

21. Verwendung von Zusammensetzungen nach einem der Ansprüche 1 bis 20 für die Herstellung von kosmetischen Zusammensetzungen zum Schutz der Haut und/oder der Haare gegen UV-Strahlung und insbesondere gegen Sonnenlicht.

22. Verwendung mindestens einer Verbindung (A) und/oder mindestens einer Verbindung (B) nach einem der Ansprüche 1 bis 8 als solubilisierendes Filtersystem, das in einer ausreichenden Menge mindestens ein in der Zusammensetzung vorliegendes, festes UV-Filter vom Typ der siliciumhaltigen Derivate mit Benzotriazolgruppe nach einem der Ansprüche 1 bis 3 alleine solubilisieren kann, für die Herstellung von kosmetischen Zusammensetzungen zum Schutz der Haut und/oder der Haare gegen UV-Strahlung.